# EUROPEAN PATENT APPLICATION

(11) **EP 4 270 411 A1**
(43) Date of publication of application: **01.11.2023**
(21) Application number: 22177525.7
(22) Date of filing: 07.06.2022
(51) Int. Cl.: G16H 30/40

(54) **ANALYSING AN ULTRASOUND IMAGE FEED**

(30) Priority: 27.04.2022 WO PCT/CN2022/089501
(71) Applicant: Koninklijke Philips N.V., 5656 AG Eindhoven (NL)
(72) Inventor: BAI, Xiang Hui, Eindhoven (NL); LIN, Qizhong, Eindhoven (NL); CHEN, Jinning, 5656AG Eindhoven (NL); SUN, Luna, 5656AG Eindhoven (NL); WANG, Crystal, Eindhoven (NL)
(74) Representative: Philips Intellectual Property & Standards

(57) **Abstract**

According to an aspect, there is provided a computer implemented method of analysing an ultrasound, US, image feed as part of an ultrasound examination. The method comprises: detecting a target tissue in a first image of the US image feed, sending a message to a display to cause the display to highlight the target tissue in the first image on the display, if the target tissue has a visibility above a first visibility threshold, and causing the display to highlight the target tissue in each consecutive US image subsequent to the first image in which the target tissue is visible in the US image feed.

## Description

### FIELD OF THE INVENTION

The disclosure herein relates to analysing an ultrasound image feed. Some embodiments herein relate to causing a display to highlight a target tissue in an ultrasound image feed.

### BACKGROUND OF THE INVENTION

There are various tools to aid clinicians (such as radiographers) to analyse and interpret ultrasound (US) image data to identify anomalies. Some tools use e.g. machine learning (ML) models to aid in the diagnostic process. For example, ML models may be used to identify particular tissues, such as lesions.

ML models may be used to identify data that may reveal abnormalities during the interpretation of patient radiological images by the clinician. For example, ML models may be used for lung nodule detection in Computed tomography (CT) images, and lesion identification in US images of breast tissue, amongst other tasks. Such models may be referred to as Computer-aided detection (CADe) models. See for example, the paper: "Computer aided detection in automated 3-D breast ultrasound images: a survey" by Kozegar et al. (2020) Artificial Intelligence Review 53, pages 1919-1941.

Although useful, there can be a complex relationship between the output of such ML models and the clinician and as such, clinical performance may depend on a variety of factors that should be considered in any study design including: the timing of the use of ML modes in the interpretive process (e.g., concurrent or second read); the physical characteristics of marks used to highlight the output of ML models on US images, e.g., size and shape, type of boundaries used to indicate bounding boxes (e.g., solid, dashed, circle, isocontour), and the proximity of such marks to the detected tissue or abnormality. Furthermore, the user's knowledge of the type of tissue that the ML model is designed to mark may affect the overall clinical performance. The number of marks provided by an ML model may also affect the manner and efficacy with which ML tools are used (for example, too many marks may confuse or distract the user, whilst too few may not convey enough information for the user to fully understand the output of the ML model).

Thus, the points above need to be carefully considered when designing and integrating ML models and their outputs into a workflow, in order ensure that the clinician is able to fully benefit from the assistance of ML models.

### SUMMARY OF THE INVENTION

Many existing workflows that incorporate ML models for analysis of medical images, such as, for example, ML models for lung nodule detection in CT images, are designed in a post-acquisition assisted reading manner. This means the images are first acquired and analysed by the ML model and the detection results are then displayed to the user (e.g. radiologist) during the interpretation stage.

However, such a workflow is not applicable for some types of examination such as e.g breast US examinations for which the image acquisition and user interpretation are inherently coupled and cannot be separated. The methods herein aim to provide improved workflows and analysis tools for US image feeds.

According to a first aspect herein, there is provided a computer implemented method of analysing an ultrasound, US, image feed as part of an ultrasound examination. The method comprises: detecting a target tissue in a first image of the US image feed; sending a message to a display to cause the display to highlight the target tissue in the first image on the display, if the target tissue has a visibility above a first visibility threshold; and causing the display to highlight the target tissue in each consecutive US image subsequent to the first image in which the target tissue is visible in the US image feed.

According to a second aspect, there is an apparatus for analysing an ultrasound, US, image feed as part of an ultrasound examination. The system comprises: a memory comprising instruction data representing a set of instructions; and a processor configured to communicate with the memory and to execute the set of instructions. The set of instructions, when executed by the processor, cause the processor to: detect a target tissue in a first image of the US image feed; send a message to a display to cause the display to highlight the target tissue in the first image on the display if the target tissue has a visibility above a first visibility threshold; and send further messages to cause the display to highlight the target tissue in each consecutive US image subsequent to the first image in which the target tissue is visible in the US image feed.

According to a third aspect there is an ultrasound, US, imaging system comprising the apparatus of the second aspect and a display to display the ultrasound image feed. The message and the further messages cause the display to highlight the target tissue in the first image on the display and in the consecutive US images.

According to a fourth aspect there is a computer program product comprising a computer readable medium, the computer readable medium having computer readable code embodied therein, the computer readable code being configured such that, on execution by a suitable computer or processor, the computer or processor is caused to perform the method of the first aspect.

Current methods of performing detection during an US examination are usually performed on an image-by-image (e.g. frame-by frame) basis. As a result, if the visibility of the target tissue changes between different US images or views in an US examination, this can result in any marks used to highlight the detected target tissue flashing on and off depending on whether the target tissue happens to appear above a detection threshold or below it in any particular image in the image feed.

In the embodiments above, the target tissue is highlighted as soon as the visibility rises above a target threshold and is further highlighted in all subsequent consecutive images thereafter. This enables the user to reliably track a detected target tissue between different images in an US image feed in which said target tissue may have variable visibility levels. There is thus provided improved methods and systems for assisting a user to analyse/check an auto-detected target tissue during a live ultrasound scan.

These and other aspects will be apparent from and elucidated with reference to the embodiment(s) described hereinafter.

### BRIEF DESCRIPTION OF THE DRAWINGS

Example embodiments will now be described, by way of example only, with reference to the following drawings, in which:
Fig. 1 shows an apparatus according to some embodiments herein;
Fig. 2 shows an US system according to some embodiments herein;
Fig. 3 shows a method according to some embodiments herein;
Fig. 4 illustrates a method according to some embodiments herein;
Fig. 5 illustrates a user interface according to some embodiments herein; and
Fig. 6 illustrates another user interface according to some embodiments herein.

### DETAILED DESCRIPTION OF EMBODIMENTS

As described above, one way of incorporating ML model detections of a target tissue into an US workflow is to display the detection results on every acquired image (e.g. frame). For example, by attaching marks, e.g., bounding boxes around the target tissue / abnormalities on each acquired image, however some problems exist with this approach.

In some cases, marks attached to the acquired images may impact the diagnosis of a user (who as noted above may be a radiologist, or other clinician) if some characteristics of the abnormality are occluded by the marks. If the abnormalities appear for a short duration in time (e.g. a low number of images in an US image feed) then the marks may be overlooked by the user. Furthermore, in many workflows that incorporate ML models, detections will be filtered using a predefined threshold, and only those detections with prediction confidence over said predefined threshold will be pushed to the user, however, the prediction confidence of the same abnormality across consecutive images varies a lot which can make the detection marks appear and then vanish, potentially multiple times, for a single abnormality across the course of an US examination. Such "flashing" marks are sub-optimal and may be distracting for the user.

In brief, what is proposed herein is a method whereby a detection of a target tissue (such as an abnormality) in a first US image above a predefined threshold is tracked between images in the US image feed and all consecutive US images to the first US image in which the target tissue is visible (even if only below the predefined threshold) are highlighted to the user. This provides continuity of detection between different views of a detection of the target tissue and also prevents the aforementioned problem of detection marks appearing and disappearing in a flashing manner.

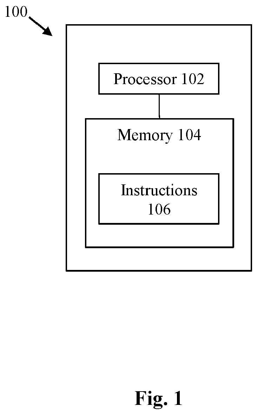

In more detail, and turning now to Fig. 1, in some embodiments there is an apparatus 100 for use in analysing an ultrasound, US, image feed, according to some embodiments herein. Generally, the apparatus may form part of a computer apparatus or system e.g. such as a laptop, desktop computer or other computing device. In some embodiments, the apparatus 100 may form part of a distributed computing arrangement or the cloud.

The apparatus comprises a memory 104 comprising instruction data representing a set of instructions and a processor 102 (e.g. processing circuitry or logic) configured to communicate with the memory and to execute the set of instructions. Generally, the set of instructions, when executed by the processor, may cause the processor to perform any of the embodiments of the method 300 as described below.

Embodiments of the apparatus 100 may be for use in analysing an ultrasound, US, image feed. More specifically, the set of instructions, when executed by the processor, cause the processor to: detect a target tissue in a first image of the US image feed; send a message to a display to cause the display to highlight the target tissue in the first image on the display, if the target tissue has a visibility above a first visibility threshold; and send further messages to cause the display to highlight the target tissue in each consecutive US image subsequent to the first image in which the target tissue is visible in the US image feed.

The processor 102 can comprise one or more processors, processing units, multi-core processors or modules that are configured or programmed to control the apparatus 100 in the manner described herein. In particular implementations, the processor 102 can comprise a plurality of software and/or hardware modules that are each configured to perform, or are for performing, individual or multiple steps of the method described herein. The processor 102 can comprise one or more processors, processing units, multi-core processors and/or modules that are configured or programmed to control the apparatus 100 in the manner described herein. In some implementations, for example, the processor 102 may comprise a plurality of (for example, interoperated) processors, processing units, multi-core processors and/or modules configured for distributed processing. It will be appreciated by a person skilled in the art that such processors, processing units, multi-core processors and/or modules may be located in different locations and may perform different steps and/or different parts of a single step of the method described herein.

The memory 104 is configured to store program code that can be executed by the processor 102 to perform the method described herein. Alternatively or in addition, one or more memories 104 may be external to (i.e. separate to or remote from) the apparatus 100. For example, one or more memories 104 may be part of another device. Memory 104 can be used to store the US image feed, the information related to the detected target tissue and/or any other information or data received, calculated or determined by the processor 102 of the apparatus 100 or from any interfaces, memories or devices that are external to the apparatus 100. The processor 102 may be configured to control the memory 104 to store the US image feed, the information related to the detected target tissue and/or any other information or data.

In some embodiments, the memory 104 may comprise a plurality of sub-memories, each sub-memory being capable of storing a piece of instruction data. For example, at least one sub-memory may store instruction data representing at least one instruction of the set of instructions, while at least one other sub-memory may store instruction data representing at least one other instruction of the set of instructions.

It will be appreciated that Fig. 1 only shows the components required to illustrate this aspect of the disclosure and, in a practical implementation, the apparatus 100 may comprise additional components to those shown. For example, the apparatus 100 may further comprise a display. A display may comprise, for example, a computer screen such as a Liquid Crystal Display (LCD), and/or a screen on a mobile phone or tablet. The apparatus may further comprise a user input device, such as a keyboard, mouse or other input device that enables a user to interact with the apparatus, for example, to provide input to be used in the methods described herein. The apparatus 100 may comprise a battery or other power supply for powering the apparatus 100 or means for connecting the apparatus 100 to a mains power supply.

In some embodiments, the apparatus is incorporated into an US imaging system. For example, an ultrasound, US, imaging system may comprise the apparatus 100 and a display to display the ultrasound image feed and the highlighting.

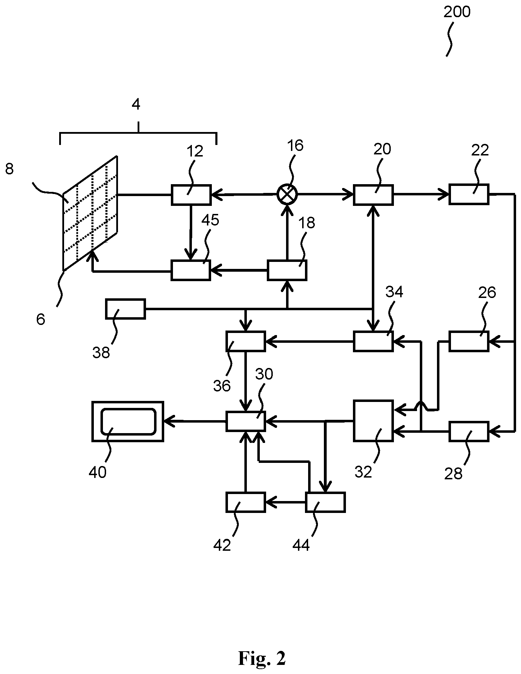

An US imaging system may further comprise other components, such as those associated with obtaining and processing US image data. An example US imaging system 200 is shown in Fig. 2. US system 200 comprises an array transducer probe 4 which has a transducer array 6 for transmitting ultrasound waves and receiving echo information. The transducer array 6 may comprise CMUT transducers; piezoelectric transducers, formed of materials such as PZT or PVDF; or any other suitable transducer technology. In this example, the transducer array 6 is a two-dimensional array of transducers 8 capable of scanning either a 2D plane or a three dimensional volume of a region of interest. In another example, the transducer array may be a 1D array.

The transducer array 6 may be coupled to a microbeamformer 12 which controls reception of signals by the transducer elements. Microbeamformers are capable of at least partial beamforming of the signals received by sub-arrays, generally referred to as "groups" or "patches", of transducers as described in US Patents 5,997,479 (Savord et al.), 6,013,032 (Savord), and 6,623,432 (Powers et al.).

In an alternative embodiment, instead of a microbeamformer 12, the transducer array may be operated directly by a main system beamformer (not shown in Fig. 2).

The system 200 may further comprise a transmit/receive (T/R) switch 16, which the microbeamformer 12 can be coupled to and which switches the array between transmission and reception modes. The transmission of ultrasound beams from the transducer array 6 is directed by a transducer controller 18 coupled to the microbeamformer by the T/R switch 16 and a main transmission beamformer (not shown), which can receive input from the user's operation of the user interface or control panel 38. The controller 18 can include transmission circuitry arranged to drive the transducer elements of the array 6 (either directly or via a microbeamformer) during the transmission mode.

It is noted that in an alternative embodiment, instead of a microbeamformer 12, the transducer array is operated directly by a main system beamformer, a T/R switch 16 may protect the main beamformer 20 from high energy transmit signals.

In a typical line-by-line imaging sequence, the beamforming system within the probe may operate as follows. During transmission, the beamformer (which may be the microbeamformer or the main system beamformer depending upon the implementation) activates the transducer array, or a sub-aperture of the transducer array. The sub-aperture may be a one dimensional line of transducers or a two dimensional patch of transducers within the larger array. In transmit mode, the focusing and steering of the ultrasound beam generated by the array, or a sub-aperture of the array, are controlled as described below.

Upon receiving the backscattered echo signals from the subject, the received signals undergo receive beamforming (as described below), in order to align the received signals, and, in the case where a sub-aperture is being used, the sub-aperture is then shifted, for example by one transducer element. The shifted sub-aperture is then activated and the process repeated until all of the transducer elements of the transducer array have been activated.

For each line (or sub-aperture), the total received signal, used to form an associated line of the final ultrasound image, will be a sum of the voltage signals measured by the transducer elements of the given sub-aperture during the receive period. The resulting line signals, following the beamforming process below, are typically referred to as radio frequency (RF) data. Each line signal (RF data set) generated by the various sub-apertures then undergoes additional processing to generate the lines of the final ultrasound image. The change in amplitude of the line signal with time will contribute to the change in brightness of the ultrasound image with depth, wherein a high amplitude peak will correspond to a bright pixel (or collection of pixels) in the final image. A peak appearing near the beginning of the line signal will represent an echo from a shallow structure, whereas peaks appearing progressively later in the line signal represent echoes from structures at increasing depths within the subject.

One of the functions controlled by the transducer controller 18 is the direction in which beams are steered and focused. Beams may be steered straight ahead from (orthogonal to) the transducer array, or at different angles for a wider field of view. The steering and focusing of the transmit beam may be controlled as a function of transducer element actuation time.

Two methods can be distinguished in general ultrasound data acquisition: plane wave imaging and "beam steered" imaging. The two methods are distinguished by a presence of the beamforming in the transmission ("beam steered" imaging) and/or reception modes (plane wave imaging and "beam steered" imaging).

Looking first to the focusing function, by activating all of the transducer elements at the same time, the transducer array generates a plane wave that diverges as it travels through the subject. In this case, the beam of ultrasonic waves remains unfocused. By introducing a position dependent time delay to the activation of the transducers, it is possible to cause the wave front of the beam to converge at a desired point, referred to as the focal zone. The focal zone is defined as the point at which the lateral beam width is less than half the transmit beam width. In this way, the lateral resolution of the final ultrasound image is improved.

For example, if the time delay causes the transducer elements to activate in a series, beginning with the outermost elements and finishing at the central element(s) of the transducer array, a focal zone would be formed at a given distance away from the probe, in line with the central element(s). The distance of the focal zone from the probe will vary depending on the time delay between each subsequent round of transducer element activations. After the beam passes the focal zone, it will begin to diverge, forming the far field imaging region. It should be noted that for focal zones located close to the transducer array, the ultrasound beam will diverge quickly in the far field leading to beam width artifacts in the final image. Typically, the near field, located between the transducer array and the focal zone, shows little detail due to the large overlap in ultrasound beams. Thus, varying the location of the focal zone can lead to significant changes in the quality of the final image.

It should be noted that, in transmit mode, only one focus may be defined unless the ultrasound image is divided into multiple focal zones (each of which may have a different transmit focus).

In addition, upon receiving the echo signals from within the subject, it is possible to perform the inverse of the above described process in order to perform receive focusing. In other words, the incoming signals may be received by the transducer elements and subject to an electronic time delay before being passed into the system for signal processing. The simplest example of this is referred to as delay-and-sum beamforming. It is possible to dynamically adjust the receive focusing of the transducer array as a function of time.

Looking now to the function of beam steering, through the correct application of time delays to the transducer elements it is possible to impart a desired angle on the ultrasound beam as it leaves the transducer array. For example, by activating a transducer on a first side of the transducer array followed by the remaining transducers in a sequence ending at the opposite side of the array, the wave front of the beam will be angled toward the second side. The size of the steering angle relative to the normal of the transducer array is dependent on the size of the time delay between subsequent transducer element activations.

Further, it is possible to focus a steered beam, wherein the total time delay applied to each transducer element is a sum of both the focusing and steering time delays. In this case, the transducer array is referred to as a phased array.

In case of the CMUT transducers, which require a DC bias voltage for their activation, the transducer controller 18 can be coupled to control a DC bias control 45 for the transducer array. The DC bias control 45 sets DC bias voltage(s) that are applied to the CMUT transducer elements.

For each transducer element of the transducer array, analog ultrasound signals, typically referred to as channel data, enter the system by way of the reception channel. In the reception channel, partially beamformed signals are produced from the channel data by the microbeamformer 12 and are then passed to a main receive beamformer 20 where the partially beamformed signals from individual patches of transducers are combined into a fully beamformed signal, referred to as radio frequency (RF) data. The beamforming performed at each stage may be carried out as described above, or may include additional functions. For example, the main beamformer 20 may have 128 channels, each of which receives a partially beamformed signal from a patch of dozens or hundreds of transducer elements. In this way, the signals received by thousands of transducers of a transducer array can contribute efficiently to a single beamformed signal.

The beamformed reception signals are coupled to a signal processor 22. The signal processor 22 can process the received echo signals in various ways, such as: band-pass filtering; decimation; I and Q component separation; and harmonic signal separation, which acts to separate linear and nonlinear signals so as to enable the identification of nonlinear (higher harmonics of the fundamental frequency) echo signals returned from tissue and micro-bubbles. The signal processor may also perform additional signal enhancement such as speckle reduction, signal compounding, and noise elimination. The band-pass filter in the signal processor can be a tracking filter, with its pass band sliding from a higher frequency band to a lower frequency band as echo signals are received from increasing depths, thereby rejecting noise at higher frequencies from greater depths that is typically devoid of anatomical information.

The beamformers for transmission and for reception are implemented in different hardware and can have different functions. Of course, the receiver beamformer is designed to take into account the characteristics of the transmission beamformer. In Fig. 2 only the receiver beamformers 12, 20 are shown, for simplicity. In the complete system, there will also be a transmission chain with a transmission micro beamformer, and a main transmission beamformer.

The function of the micro beamformer 12 is to provide an initial combination of signals in order to decrease the number of analog signal paths. This is typically performed in the analog domain.

The final beamforming is done in the main beamformer 20 and is typically after digitization.

The transmission and reception channels use the same transducer array 6 which has a fixed frequency band. However, the bandwidth that the transmission pulses occupy can vary depending on the transmission beamforming used. The reception channel can capture the whole transducer bandwidth (which is the classic approach) or, by using bandpass processing, it can extract only the bandwidth that contains the desired information (e.g. the harmonics of the main harmonic).

The RF signals may then be coupled to a B mode (i.e. brightness mode, or 2D imaging mode) processor 26 and a Doppler processor 28. The B mode processor 26 performs amplitude detection on the received ultrasound signal for the imaging of structures in the body, such as organ tissue and blood vessels. In the case of line-by-line imaging, each line (beam) is represented by an associated RF signal, the amplitude of which is used to generate a brightness value to be assigned to a pixel in the B mode image. The exact location of the pixel within the image is determined by the location of the associated amplitude measurement along the RF signal and the line (beam) number of the RF signal. B mode images of such structures may be formed in the harmonic or fundamental image mode, or a combination of both as described in US Pat. 6,283,919 (Roundhill et al.) and US Pat. 6,458,083 (Jago et al.) The Doppler processor 28 processes temporally distinct signals arising from tissue movement and blood flow for the detection of moving substances, such as the flow of blood cells in the image field. The Doppler processor 28 typically includes a wall filter with parameters set to pass or reject echoes returned from selected types of materials in the body.

The structural and motion signals produced by the B mode and Doppler processors are coupled to a scan converter 32 and a multi-planar reformatter 44. The scan converter 32 arranges the echo signals in the spatial relationship from which they were received in a desired image format. In other words, the scan converter acts to convert the RF data from a cylindrical coordinate system to a Cartesian coordinate system appropriate for displaying an ultrasound image on an image display 40. In the case of B mode imaging, the brightness of pixel at a given coordinate is proportional to the amplitude of the RF signal received from that location. For instance, the scan converter may arrange the echo signal into a two dimensional (2D) sector-shaped format, or a pyramidal three dimensional (3D) image. The scan converter can overlay a B mode structural image with colors corresponding to motion at points in the image field, where the Doppler-estimated velocities to produce a given color. The combined B mode structural image and color Doppler image is able to depict tissue motion and blood flow within the structural image field. The multi-planar reformatter will convert echoes that are received from points in a common plane in a volumetric region of the body into an ultrasound image of that plane, as described in US Pat. 6,443,896 (Detmer). A volume renderer 42 converts the echo signals of a 3D data set into a projected 3D image as viewed from a given reference point as described in US Pat. 6,530,885 (Entrekin et al.).

The 2D or 3D images are coupled from the scan converter 32, multi-planar reformatter 44, and volume renderer 42 to an image processor 30 for further enhancement, buffering and temporary storage for display on an image display 40. The imaging processor may be adapted to remove certain imaging artifacts from the final ultrasound image, such as for example,: acoustic shadowing, for example caused by a strong attenuator or refraction; posterior enhancement, for example caused by a weak attenuator; reverberation artifacts, for example where highly reflective tissue interfaces are located in close proximity; and so on. In addition, the image processor may be adapted to handle certain speckle reduction functions, in order to improve the contrast of the final ultrasound image.

In addition to being used for imaging, the blood flow values produced by the Doppler processor 28 and tissue structure information produced by the B mode processor 26 are coupled to a quantification processor 34. The quantification processor may be used for making measurements in the images. The quantification processor may receive input from a user control panel 38.

Output data from the quantification processor is coupled to a graphics processor 36 for the reproduction of measurement graphics and values with the image on the display 40, and for audio output from the display device 40. The graphics processor 36 can also generate graphic overlays for display with the ultrasound images. These graphic overlays can contain standard identifying information such as patient name, date and time of the image, imaging parameters, and the like. For these purposes the graphics processor receives input from the user interface 38, such as patient name. The user interface is also coupled to the transmit controller 18 to control the generation of ultrasound signals from the transducer array 6 and hence the images produced by the transducer array and the ultrasound system. The transmit control function of the controller 18 is only one of the functions performed. The controller 18 also takes account of the mode of operation (given by the user) and the corresponding required transmitter configuration and band-pass configuration in the receiver analog to digital converter. The controller 18 can be a state machine with fixed states.

The user interface is also coupled to the multi-planar reformatter 44 for selection and control of the planes of multiple multi-planar reformatted (MPR) images which may be used to perform quantified measures in the image field of the MPR images.

It will be appreciated that the US image system illustrated in Fig 2 is merely an example and that an US image system may comprise different components to those described above.

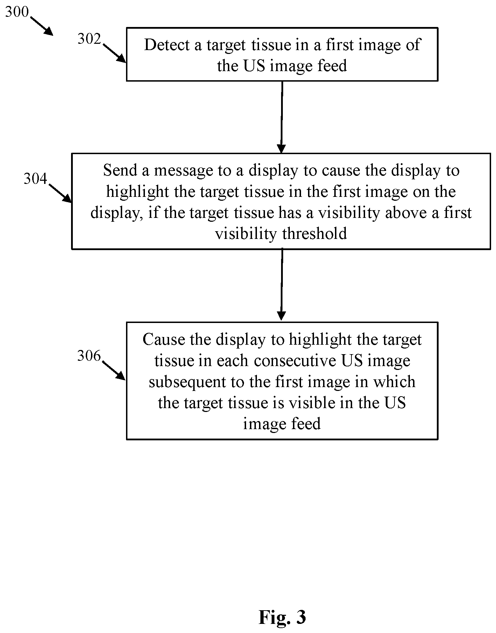

Turning now to Fig. 3, there is a computer implemented method 300 for use in analysing an US image feed. Embodiments of the method 300 may be performed, for example by an apparatus such as the apparatus 100 or the US imaging system 200 described above.

The method 300 may be performed to assist a user such as a clinician or radiographer to perform an US examination on a subject to identify a target tissue in the subject.

Briefly, in a first step 302, the method 300 comprises: detecting a target tissue in a first image of the US image feed. In a second step 304 the method 300 comprises sending a message to a display to cause the display to highlight the target tissue in the first image on the display, if the target tissue has a visibility above a first visibility threshold. In a third step the method 300 comprises causing the display to highlight the target tissue in each consecutive US image subsequent to the first image in which the target tissue is visible in the US image feed.

In more detail, an US image feed comprises a plurality of US images (or image frames). The US image feed may be a real-time (or near real-time) feed. In other words, the US image feed may be a live US image feed, e.g. one that is in the process of being obtained as part of an US examination. The US image feed may be obtained using an US system such as the system 200 illustrated in Fig 2.

In step 302 the method comprises detecting a target tissue in a first image of the US image feed. As used herein, the target tissue, or target tissue type, may be any tissue of interest. For example, an abnormal tissue or abnormality, such as a lesion, or a specific type of lesion. In other embodiments the target tissue could be normal tissue. Generally, the US image feed is obtained in order to determine whether the target tissue is present, or absent in the subject of the ultrasound examination.

The target tissue may be detected using any known technique, such as, for example, an image processing technique. The target tissue may be detected using a Computer-aided detection (CADe) model.

In some embodiments, Machine Learning techniques may be used to detect the target tissue in step 302. For example, the step of detecting a target tissue in a first image of the US image feed may be performed using a machine learning model trained to detect target tissues in US images. For example, a machine learning model that was trained using training data comprising training examples, each training example comprising: an example US image and a corresponding correct detection(s) of the target tissue in the example US image (e.g. example inputs and example ground truth outputs).

The skilled person will be familiar with machine learning (ML) models that may be trained to detect features such as particular type of target tissue in US images. For example, a convolutional neural network may be trained to detect a target tissue in an US image, using training data comprising example US image(s) and example ground truth (e.g. "correct") annotations of whether the respective example US image(s) contain the target tissue type.

As an example, a You Only Look Once (YOLO) network described in the paper by Redmon et al. (2016) entitled "You Only Look Once: Unified, Real-Time Object Detection*"* may be trained to detect a target tissue in US images in real-time in US image feeds.

As another example, a U-net network described in paper "U-Net: Convolutional Networks for Biomedical Image Segmentation" by Olaf Ronneberger et al. can be trained to segment out the target tissue from an US image feed.

The skilled person will appreciate however that this is merely an example, and that there are many ways that a target tissue may be detected in US images and US image feeds, for example, using techniques such as Radial Gradient Index (RGI) Filtering, Multifractal Filtering, Rule-based Region Ranking and/or deformable Part Models.

The output of step 302 may be an indication of one or more detections of the target tissue accompanied by a respective visibility for each detection. In this sense a visibility may be, e.g. a brightness level or score associated with the detection; a confidence level associated with the detection or any other measure that may be used as an indication of whether the detection of the target tissue is a real detection (or a false positive detection).

In embodiments where step 302 is performed using machine learning, the visibility may be based on a confidence score (e.g. probability score) output by the model to indicate the probability that the output of the model is correct.

The detection technique may be performed on each US image in the US image feed (e.g. in sequence).

In step 304, if the target tissue (e.g. the target tissue detected in step 302) has a visibility above a first visibility threshold, then the method 300 comprises sending a message to a display to cause the display to highlight the target tissue in the first image on the display. In other words, the first US image is the first US image in the sequence that contains a detection with a visibility above the first visibility threshold and this detection acts as a trigger to highlight the detected target tissue in the first US image.

The first visibility threshold may be, for example, a threshold brightness level. In embodiments where the detection in step 302 is performed using machine learning, then the first visibility threshold may be a threshold confidence level in a detection of the target tissue. In other words, step 304 may be performed if (or in response to) detecting the target tissue with a confidence above the threshold confidence level.

The target tissue may be highlighted on the display in any manner. For example, using a bounding box or bounding circle, a text string or alert, a symbol, using colour to highlight (or fill) a region comprising the detected target tissue, or using any other mark that may draw attention to the detected target tissue. In some embodiments, where the US image feed is a live, or real-time image feed, the message may further cause the display to display an alert that a live detection has occurred.

In step 306 the method then comprises causing the display to highlight the target tissue in each consecutive US image, subsequent to the first image, in which the target tissue is visible in the US image feed.

Step 306 may comprise tracking the target tissue in US images subsequent to the first image in the ultrasound image feed. E.g., the target tissue may be tracked between consecutive US images in the US image feed and highlighted in each subsequent US image to the first US image in which the target tissue is detected. Thus the target tissue is highlighted in a sequence of images in which the target tissue is consistently detected and trackable between one image of the sequence and the next.

The skilled person will be familiar with methods of tracking an object from one image in a sequence of images to another. For example, an object may be tracked from one image in a sequence to another if the centres of detections in the respective images are within a predefined range (in other words if they appear in approximately the same position in consecutive images).

In some embodiments, the step of causing the display to highlight the target tissue in each consecutive US image subsequent to the first image in which the target tissue is visible in the US image feed, is performed even if in one or more consecutive US image the target tissue is visible with a visibility below the first visibility threshold.

For example, the target tissue may be highlighted in every consecutive image in which a detection is made above a second visibility threshold, the second visibility threshold being lower than the first visibility threshold.

In embodiments where the target tissue is detected using ML, the step of causing the display to highlight the target tissue in each consecutive US image subsequent to the first image in which the target tissue is visible in the US image feed, may be performed even if in one or more consecutive US image the target tissue is detected by the ML model with a confidence below the threshold confidence level.

In this way a detection of a target tissue may be tracked and highlighted between US images in an US image feed, even if the confidence or visibility changes between the images. Visibility may change, for example, due to viewing angle, or viewing depth, but using the method 300 herein, so long as a detection is made in a first US image above a threshold visibility, the target tissue is highlighted in a continuous manner to aid the user to track the target tissue through different US images in the US image feed.

In some embodiments, a display may display the US image feed in both a primary window of the display and a secondary window of the display. The step of highlighting the target tissue in the first image on the display may thus be performed in the US image feed displayed in the secondary window. This is illustrated in Fig, 5 below where the primary window corresponds to the Main Display region and the secondary window corresponds to the Live Detection region.

Turning now to other embodiments, in some embodiments, the method 300 may further comprise grouping the first image and the consecutive US images subsequent to the first image in which the target tissue is visible, as a first group of images.

Furthermore, the target tissue may be tracked in one or more consecutive US images directly preceding the first US image in which the target tissue is visible (e.g below the first visibility threshold) and these may also be added to the first group of images. In this way, although a detection above the first visibility threshold triggers highlighting of the target tissue, a sequence of US images in the US image feed in which the target tissue is continuously visible may be grouped to form the first group of images. The first group of images thus form a clip or extract from the full US image feed.

The first group of images may be stored for later retrieval and viewing by the user.

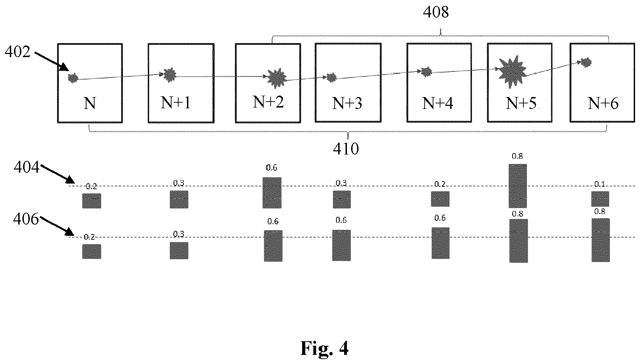

This is illustrated in Fig 4 which shows an embodiment of the method 300. In this embodiment, there is an US image feed comprising images N to N+6. US images N to N+6 show a target tissue 402. In step 302 of the method 300 in this embodiment, the target tissue 402 is detected using a ML model. The target tissue 402 is detectable above a first threshold confidence level (indicated by the dashed line 404) by the ML model in a first US image, labelled N+2 in Fig. 4. The target tissue is detected in consecutive subsequent images N+3-N+6 (images 408), and these are highlighted, even though in images N+3, N=4 and N+6 the target tissue is detected with a confidence below the first threshold confidence 404.

The target tissue is also detected in images N and N+1 and these are added to the images 408 to form a first group of images 410 (e.g. a clip) that can be played back to the user on request.

Thus in this way, the detections on consecutive images are linked into a group (the linking method is not limited, e.g., the centres of two consecutive detections markers are within a predefined range) and the max prediction confidence of the detections in this group will be used as the prediction confidence of the group. With this setting, during an US scan, once a detection is beyond the first threshold confidence level 404, the detections on subsequent consecutive images will be all pushed to user and visible in the live detection region. Furthermore, as described below, if this target tissue detection is saved in a detection buffer, all the images containing detections in the first group of images 410 can be all saved together.

In some embodiments, the US image feed is buffered, for example, a predetermined interval of the US image feed may be held in a buffer (e.g. the last x minutes may be buffered, irrespective of the image content) and upon receiving a request from a user, a portion of the buffered US image feed may be replayed to the user, with the target tissue in the first group of images highlighted in the replay.

In some embodiments, when the user starts to replay a stored group of images (e.g. a stored clip), image acquisition may be paused and the buffer freezed and stored for the user to replay, and the time period corresponding to the stored group of images/clip may be marked on a time bar (e.g. in the manner of a slide bar). In other words, upon receiving a request from a user, the display may be caused to replay a portion of the buffered US image feed and highlight a time period in the portion of the buffered US image feed on a time bar that corresponds to the (location in time of the) first group of images. This may help the user to quickly jump to these images for further review.

Upon resumption of the US scanning, the buffer may be cleared since all those worth checking detections have been reviewed by the user.

In this way, a user may easily locate a detection in a buffered US image feed and replay portions of the US image feed to review both the feed and the output of an automated detection process in order to reassess a detection.

It will be appreciated that the method 300 described above may be performed for more than one target tissue detection in each US image and may be repeated for different target tissue detections in different parts of the US images in the US image feed.

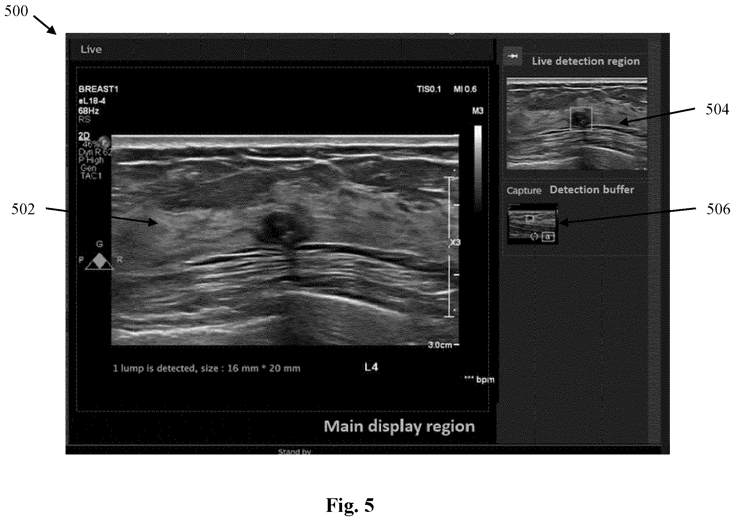

Turning now to Fig. 5 which illustrates a user interface 500 according to some embodiments herein. In this embodiment there are three areas: Main display region 502, Live detection region (or mirror window) 504 and a Detection buffer 506. In this embodiment, the acquired US image feed will be displayed on the main display region 502 without detection markers attached to it. Once a target tissue is detected, a notification message is displayed on the bottom of the Main display region 502 to prompt the user there is a detection on this image. At the same time, markers pointing to the detection are displayed on the live detection region 504 and all the consecutive images containing this target tissue will be auto saved in the detection buffer. If the user has already noticed the target tissue, they can just ignore this message and continue scanning. Otherwise, they can check the live detection markers or review the latest detection on the detection buffer 506 to quickly confirm whether this detection is worth further reviewing further.

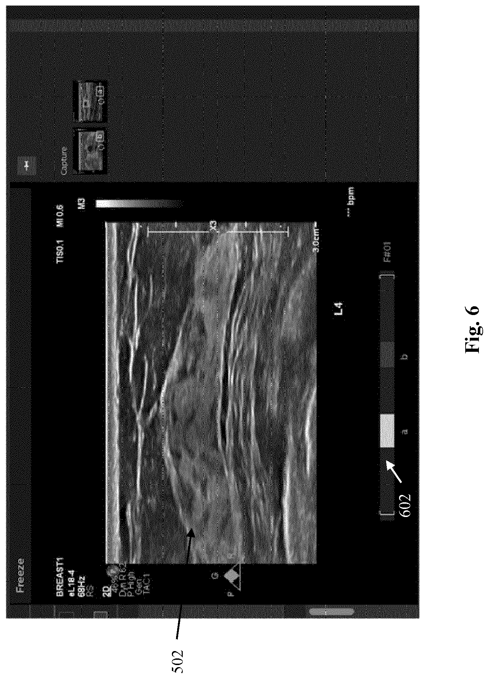

Fig. 6 illustrates another user interface that may be used in connection with the user interface 500 described above. If the user decides to further review the detection in Fig. 5, they can press a "freeze" button on the ultrasound machine to pause the image acquisition. At this point, several hundreds of acquired images (e.g. the images acquired right before "freeze" was pressed) will be saved in a frame buffer and displayed on the main display region 502. The user interface may have a slide bar (e.g time bar) 602 on the bottom, in which the group(s) of images with detections are highlighted (e.g. in a different colour) to help the user to quickly jump to these frames for further review. As noted above, the user can press the "freeze" button again to quit the review mode and continue scanning, after that the detection buffer may be cleared since all those worth checking detections have been reviewed by the user.

Turning to other embodiments, in another embodiment, there is provided a computer program product comprising a computer readable medium, the computer readable medium having computer readable code embodied therein, the computer readable code being configured such that, on execution by a suitable computer or processor, the computer or processor is caused to perform the method or methods described herein.

Thus, it will be appreciated that the disclosure also applies to computer programs, particularly computer programs on or in a carrier, adapted to put embodiments into practice. The program may be in the form of a source code, an object code, a code intermediate source and an object code such as in a partially compiled form, or in any other form suitable for use in the implementation of the method according to the embodiments described herein.

It will also be appreciated that such a program may have many different architectural designs. For example, a program code implementing the functionality of the method or system may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A computer implemented method of analysing an ultrasound, US, image feed as part of an ultrasound examination, the method comprising:
detecting (302) a target tissue in a first image of the US image feed;
sending (304) a message to a display to cause the display to highlight the target tissue in the first image on the display, if the target tissue has a visibility above a first visibility threshold; and
c ausing (306) the display to highlight the target tissue in each consecutive US image subsequent to the first image in which the target tissue is visible in the US image feed.

2. A method as in claim 1 wherein the step of causing (306) the display to highlight the target tissue in each consecutive US image subsequent to the first image in which the target tissue is visible in the US image feed, is performed even if in one or more consecutive US image the target tissue is visible with a visibility below the first visibility threshold.

3. A method as in claim 1 or 2 wherein the step of detecting (302) a target tissue in a first image of the US image feed is performed using a machine learning model trained to detect target tissues in US images.

4. A method as in claim 3 wherein the first visibility threshold is a threshold confidence level, and the step of: sending (304) a message to a display to cause the display to highlight the target tissue in the first image on the display, is performed in response to the machine learning model detecting the target tissue with a confidence above the threshold confidence level.

5. A method as in any one of the preceding claims wherein the step of causing (306) the display to highlight the target tissue in each consecutive US image, subsequent to the first image, in which the target tissue is visible in the US image feed comprises:
tracking the target tissue in US images subsequent to the first image in the ultrasound image feed.

6. A method as in any one of the preceding claims wherein the method further comprises: grouping the first image and the consecutive US images subsequent to the first image in which the target tissue is visible, as a first group of images.

7. A method as in claim 6 wherein the first group of US images further comprises consecutive US images directly preceding the first image in which the target tissue is visible below the first visibility threshold.

8. A method as in claim 6 or 7 further comprising:
buffering the US image feed;
and upon receiving a request from a user, causing the display to replay a portion of the buffered US image feed and highlight a time period in the portion of the buffered US image feed on a time bar that corresponds to the first group of images.

9. A method as in any one of the preceding claims, wherein the method is performed in real-time on a live ultrasound image feed.

10. A method as in claim 9 wherein the step of: sending (304) a message to a display, causes the display to display an alert that a live detection has occurred.

11. A method as in any one of the preceding claims further comprising causing the display to:
display the US image feed in a primary window of the display;
display the US image feed in a secondary window of the display; and wherein the step of highlighting the target tissue in the first image on the display is performed in the US image feed displayed in the secondary window.

12. A method as in any one of the preceding claims wherein the US image feed comprises images of breast tissue obtained as part of a breast lesion examination.

13. An apparatus for analysing an ultrasound, US, image feed as part of an ultrasound examination, the system comprising:
a memory comprising instruction data representing a set of instructions; and
a processor configured to communicate with the memory and to execute the set of instructions, wherein the set of instructions, when executed by the processor, cause the processor to:
detect a target tissue in a first image of the US image feed;
send a message to a display to cause the display to highlight the target tissue in the first image on the display, if the target tissue has a visibility above a first visibility threshold; and
send further messages to cause the display to highlight the target tissue in each consecutive US image subsequent to the first image in which the target tissue is visible in the US image feed.

14. An ultrasound, US, imaging system comprising the apparatus of claim 13 and a display to display the ultrasound image feed; and wherein the message and the further messages cause the display to highlight the target tissue in the first image on the display and in the consecutive US images.

15. A computer program product comprising a computer readable medium, the computer readable medium having computer readable code embodied therein, the computer readable code being configured such that, on execution by a suitable computer or processor, the computer or processor is caused to perform the method as claimed in any one of claims 1 to 12.
